(19) Europäisches Patentamt
European Patent Office
Office européen des brevets

(11) Veröffentlichungsnummer : **0 027 908**
**B1**

(12) **EUROPÄISCHE PATENTSCHRIFT**

(45) Veröffentlichungstag der Patentschrift : **14.09.83**

(21) Anmeldenummer : **80105893.4**

(22) Anmeldetag : **29.09.80**

(51) Int. Cl.³ : **C 07 D211/46**, C 07 D211/54, C 07 H 9/04, C 07 H 15/12, A 61 K 31/445// C07D498/04

# (54) 2-Hydroxyalkyl-3,4,5-trihydroxypiperidine, Verfahren zu ihrer Herstellung und ihre Verwendung als Arzneimittel.

(30) Priorität : **19.10.79 DE 2942365**

(43) Veröffentlichungstag der Anmeldung : **06.05.81 Patentblatt 81/18**

(45) Bekanntmachung des Hinweises auf die Patenterteilung : **14.09.83 Patentblatt 83/37**

(84) Benannte Vertragsstaaten : **AT BE CH DE FR GB IT LI SE**

(56) Entgegenhaltungen :
**EP A 0 000 947**
**DE A 2 915 037**
**DE B 1 768 044**
**DE B 2 824 781**

**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Kinast, Günther, Dr.**
**Am Eckbusch 35/33**
**D-5600 Wuppertal-1 (DE)**
Erfinder : **Müller, Lutz, Dr.**
**Kronprinzenallee 111**
**D-5600 Wuppertal-1 (DE)**
Erfinder : **Sitt, Rüdiger, Dr.**
**Claudiusweg 9**
**D-5600 Wuppertal-1 (DE)**
Erfinder : **Puls, Walter, Dr.**
**In den Birken 75**
**D-5600 Wuppertal-1 (DE)**

**EP 0 027 908 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# 2-Hydroxyalkyl-3,4,5-trihydroxypiperidine, Verfahren zu ihrer Herstellung und ihre Verwendung als Arzneimittel

Die Erfindung betrifft neue Derivate des 2-Hydroxyalkyl-3,4,5-trihydroxypiperidins, Verfahren zu ihrer Herstellung sowie ihre Verwendung als Arzneimittel, insbesondere gegen Diabetes, Hyperlipoproteinämie, Atherosklerose und Adipositas.

Es ist bereits bekannt geworden, daß N-Alkyl- und N-Alkenyl-Derivate des 2-Hydroxymethyl-3,4,5-trihydroxy-piperidins potente Inhibitoren für α-Glukosidhydrolasen sind (Europäische Offenlegungsschrift 947).

Es wurden nun neue Derivate des 2-Hydroxyalkyl-3,4,5-trihydroxypiperidins gefunden, die eine verstärkte Wirkung auf α-Glukosidhydrolasen aufweisen. Die neuen Verbindungen entsprechen der allgemeinen Formel I

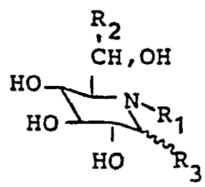

(I)

worin

$R_1$ Wasserstoff,

$R_2$ gegebenenfalls durch Hydroxy, $C_1$-$C_4$-Alkoxy oder Phenyl, das seinerseits durch $C_1$-$C_4$-Alkyl, $C_1$-$C_4$-Alkoxy, Halogen, Nitro oder Cyan substituiert sein kann, substituiertes $C_1$-$C_{10}$-Alkyl, $C_2$-$C_6$-Alkenyl, $C_4$-$C_{10}$-Alkandienyl oder gegebenenfalls durch $C_1$-$C_4$-Alkyl, $C_1$-$C_4$-Alkoxy, Halogen, Nitro oder Cyan substituiertes Phenyl und

$R_3$ Wasserstoff, Sulfo oder Hydroxy bedeuten.

Verbindungen der allgemeinen Formel I erhält man, wenn man Verbindungen der allgemeinen Formel II

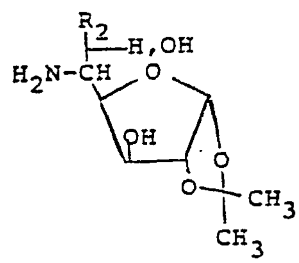

(II)

worin $R_2$ die oben genannte Bedeutung hat, mit Schwefeldioxid umsetzt, die entstehenden Verbindungen der allgemeinen Formel I, worin $R_3$ $SO_3H$ bedeutet, gegebenenfalls mit Basen oder basischen Ionenaustauschern zu Verbindungen der allgemeinen Formel I, worin $R_3$ OH bedeutet, umsetzt, und diese gegebenenfalls zu Verbindungen der allgemeinen Formel I, worin $R_3$ Wasserstoff bedeutet, reduziert.

Die Verbindungen der allgemeinen Formel II werden z. B. nach folgendem Reaktionsschema gewonnen :

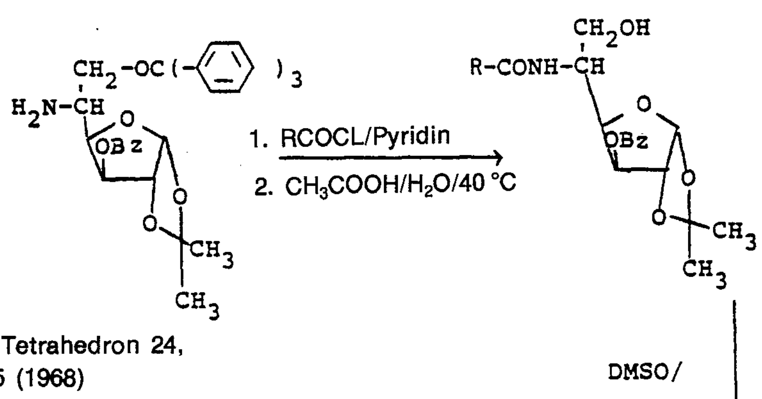


vgl. Tetrahedron 24, 2125 (1968)

R=$CH_3$ oder BzO—

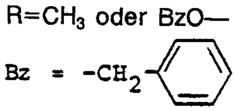

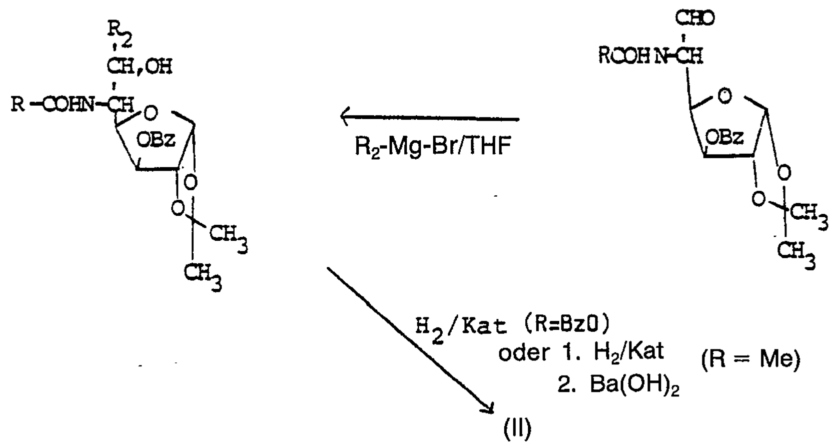


(III)

(II)

Für die Aminogruppe kann z. B. Benzyloxycarbonyl oder Acetyl als Schutzgruppe verwendet werden. Die Verbindungen der allgemeinen Formel III, in denen R für $CH_3$ oder BzO— steht, stellen wichtige Zwischenverbindungen zur Herstellung der erfindungsgemäßen Verbindungen I dar.

Weitere Einzelheiten sind in den Ausführungsbeispielen beschrieben.

Die Umsetzung von II mit $SO_2$ unter Abspaltung der Isopropylidengruppe und Bildung des Piperidinringes wird so durchgeführt, daß man die wäßrige oder wasserhaltige alkoholische Lösung der Verbindungen der allgemeinen Formel II mit $SO_2$ sättigt und mehrere Tage bei Temperaturen zwischen 20 °C und 50 °C aufbewahrt. Die Verbindungen der allgemeinen Formel I fallen dann als meist gut kristallisierende Bisulfitaddukte ($R_3$=—$SO_3H$) an, aus denen sich die Verbindungen der allgemeinen Formel I ($R_3$=—OH) mit Hilfe von z. B wäßrigem $Ba(OH)_2$ freisetzen lassen.

Die Reduktion von Verbindungen der allgemeinen Formel I mit $R_3$=OH zu Verbindungen der allgemeinen Formel I mit $R_3$=H erfolgt durch Verwendung von Alkalimetallborhydriden, Alkalimetallcyanoborhydriden oder auch von Dialkylaminoboranen. Bevorzugt ist die Verwendung von Natriumcyanoborhydrid in wäßriger Lösung oder in einem mit Wasser mischbaren wasserhaltigen organischen Lösungsmittel, wie beispielsweise Methanol bei Raumtemperatur oder gegebenenfalls erhöhter Temperatur. Ganz besonders bevorzugt erfolgt die Reduktion jedoch katalytisch mit Pt oder Pd als Katalysator oder in Gegenwart von Raney-Ni. Dabei arbeitet man bevorzugt in wäßriger Lösung bei Raumtemperatur.

Ein weiteres Verfahren zur Herstellung der erfindungsgemäßen Verbindungen der allgemeinen Formel I in der $R_3$=H ist, läßt sich durch folgendes Reaktionsschema darstellen :

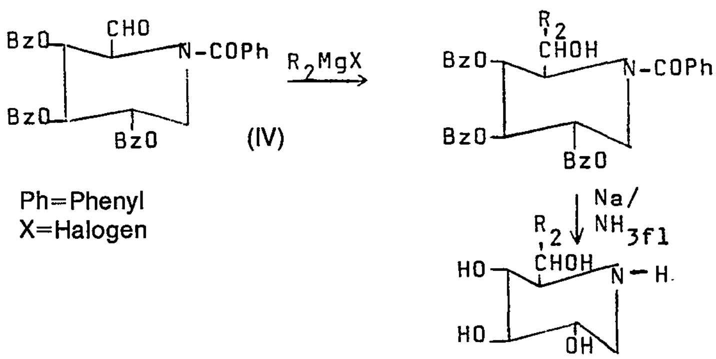


(IV)

Ph=Phenyl
X=Halogen

Die Herstellung des eingesetzten Aldehyds IV kann z. B. durch Oxidation des entsprechenden Alkohols mit Dicyclohexylcarbodiimid in DMSO in Gegenwart von Phosphorsäure erfolgen.

Die Herstellung des entsprechenden Alkohols ist im Beispiel 3 beschrieben.

Zwei Verfahren zur Herstellung der Verbindung IV lassen sich durch die folgenden Formelschemata beschreiben

(1)

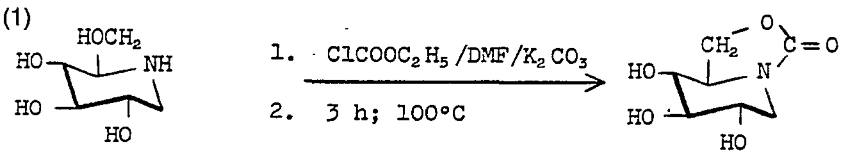

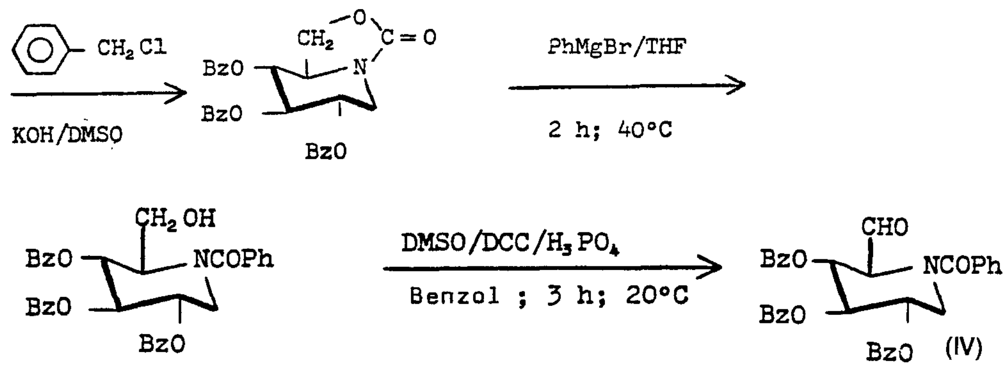


(IV)

(2)

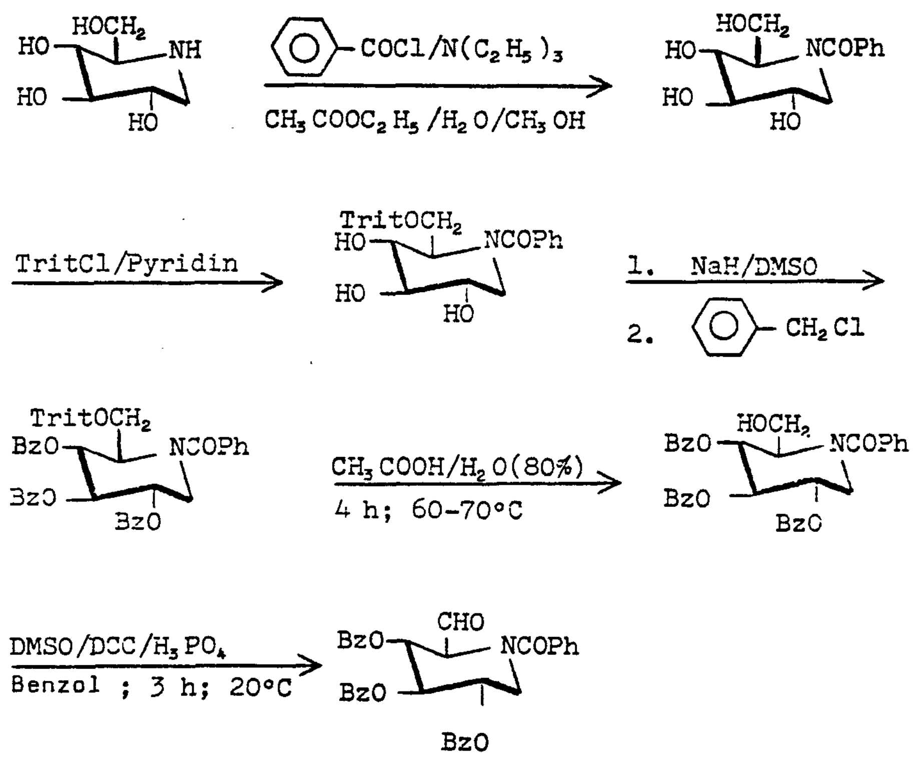


(IV)

Verbindung IV, der korrespondierende Alkohol und N, 7-0-Cyclocarbamato-2-hydroxymethyl-3,4,5-trihydroxypiperidin sind wichtige Zwischenprodukte für die Herstellung der Verbindungen I.

Bevorzugt bedeutet $R_2$ $C_1$-$C_8$-Alkyl, Allyl, Benzyl oder Phenyl und $R_3$ Wasserstoff, Sulfo oder Hydroxy.

In ganz besonders bevorzugten Verbindungen der allgemeinen Formel I bedeutet $R_2$ Methyl oder Ethyl und $R_3$ Wasserstoff bzw. $R_2$ Methyl und $R_3$ Sulfo oder Hydroxy.

Die erfindungsgemäßen Inhibitoren eignen sich als Therapeutika für folgende Indikationen bei Mensch und Tier:

Prädiabetes, Gastritis, Obstipation, Karies, Infektionen des Gastro-Intestinaltraktes, Meteorismus, Flatulenz, Hypertension, und besonders Atherosklerose, Adipositas, Diabetes und Hyperlipoproteinämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationen der erfindungsgemäßen Inhibitoren mit bereits bekannten handelt.

Vorteilhaft sind in machen Fällen auch Kombinationen der erfindungsgemäßen Inhibitoren mit bekannten oralen Antidiabetika (β-cytotrope Sulfonylharnstoffderivate und/oder blutzuckerwirksame Biguanide) sowie mit blutlipid-senkenden Wirkstoffen wie z. B. Clofibrat, Nicotinsäure, Cholestyramin und anderen.

Die Verbindungen können ohne Verdünnung, z. B. als Pulver oder in einer Gelatinehülle oder in Kombination mit einem Trägerstoff in einer pharmazeutischen Zusammensetzung appliziert werden.

Pharmazeutische Zubereitungen können eine größere oder kleinere Menge des Inhibitors enthalten, z. B. 0,1 % bis 99,5 %, in Kombination mit einem pharmazeutisch verträglichen nicht-toxischen, inerten Trägerstoff, wobei der Trägerstoff eine oder mehrere feste, halbfeste oder flüssige Verdünnungsmittel, Füllstoffe und/oder nicht-toxische, inerte und pharmazeutischverträgliche Formulierungshilfsmittel enthalten kann. Solche pharmazeutischen Zubereitungen liegen vorzugsweise in Form von Dosierungseinheiten vor, d.h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder 1/2, 1/3 oder 1/4 einer Einzeldosis enthalten. Eine Einzeldosis enthält vorzugsweise eine genügende Menge Wirkstoff, um bei einer Applikation gemäß eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen Haupt- und Nebenmahlzeiten am Tage verabreicht wird.

Andere therapeutische Mittel können auch eingenommen werden. Obgleich die Dosierung und das Dosierungsschema in jedem Fall sorgsam abgewogen werden sollte, unter Anwendung gründlichen fachmännischen Urteils und unter Beachtung des Alters, des Gewichts und des Zustands des Patienten, der Art und der Schwere der Erkrankung, wird die Dosierung gewöhnlich in einem Bereich zwischen etwa 0,05 bis etwa 10 mg/kg des Körpergewichtes pro Tag liegen. In manchen Fällen wird man dabei eine ausreichende therapeutische Wirkung mit einer geringeren Dosis erreichen, während in anderen Fällen eine größere Dosis erforderlich sein wird.

## Herstellungsbeispiele

1. a) 5-Acetamido-3-O-benzyl-5-desoxy-1,2-O-isopropyliden-6-O-triphenylmethyl-α-D-glucofuranose

551 g 5-Amino-5-desoxy-3-O-benzyl-1,2-O-isopropyliden-6-O-triphenylmethyl-α-D-glucofurannose [S. Inouye, T. Tsurnoka, T. Ito und T. Niida, Tetrahedron 24, 2125-2144 (1968)], 400 ml Dichlormethan, 400 ml Pyridin und 200 ml Acetanhydrid werden bei 0-20 °C zusammengegeben und 24 Stunden bei Raumtemperatur gerührt. Dann wird das Methylenchlorid im Vakuum abgezogen, der Rückstand mit 300 g Eis versetzt und 30 Minuten gerührt. Das Gemisch wird dreimal mit je 300 ml Chloroform extrahiert, der Extrakt zweimal mit Wasser und zweimal mit Natriumhydrogencarbonat-Lösung gewaschen, über Natriumsulfat getrocknet und im Vakuum bei 40 °C Badtemperatur eingeengt. Ausbeute : 600 g.

b) 5-Acetamido-3-O-benzyl-5-desoxy-1,2-O-isopropyliden-α-D-glucofuranose

600 g rohe 5-Acetamido-5-desoxy-3-O-benzyl-1,2-O-isopropyliden-6-O-triphenylmethyl-α-D-glucofuranose werden in 1,5 ml Eisessig gelöst mit 600 ml Wasser versetzt, 2 h bei 70 °C und über Nacht bei 20 °C gerührt. Die Umsetzung wird dünnschichtchromatographisch verfolgt (Chloroform/Essigester 3 : 1 und Chloroform/Methanol 10 : 1). Der Niederschlag wird abgesaugt, mit Eisessig/Wasser 1 : 1 gewaschen und verworfen. Die vereinigten Filtrate werden bei 50 °C im Vakuum eingedampft. Der Rückstand wird in 1 l Essigsäureethylester aufgenommen, unlösliche Bestandteile werden abfiltriert, die Essigesterphase wird mit Wasser und mit Natriumhydrogencarbonat-Lösung neutral gewaschen, über Natriumsulfat getrocknet und im Vakuum eingedampft. Das erhaltene Öl kristallisiert aus Methanol/Wasser, Ausbeute 101 g vom Schmp. 84-88 °C. Die Mutterlauge wird eingedampft, in wenig Ether aufgenommen auf eine Kieselgel-Säule gegeben und nacheinander mit 6 l Ether, 5 l Essigester und 2,5 l Methanol eluiert. Das Ether-Eluat wird verworfen, das Essigester- und das Methanol-Eluat wird jeweils eingedampft und der Rückstand aus Methanol/Wasser kristallisiert. Insgesamt erhält man : 214 g (61 %), vom Schmp. 84-88 °C.

c) 5-Acetamido-3-O-benzyl-5-desoxy-1,2,O-iso-propyliden-α-D-glucofuranose

51 g 5-Acetamido-6-O-acetyl-3-O-benzyl-5-desoxy-1,2-O-isopropyliden-α-D-glucofuranose [H. Saeki et al., Chem. Pharm. Bull 26, 2477 (1968)], 160 ml Methanol und 0,2 g Natriummethylat werden über Nacht bei Raumtemperatur gerührt. Dann wird der Ansatz mit $CO_2$ (Trockeneis) neutralisiert, im Vakuum eingedampft, der Rückstand in Essigsäureethylester aufgenommen, zweimal mit Wasser gewaschen, über Natriumsulfat getrocknet, im Vakuum eingedampft und der Rückstand aus Ether/Petrolether kristallisiert. Ausbeute 43 g (94 % der Theorie) vom Schmp. 88 °C.

d) 5-Acetamido-3-O-benzyl-5-desoxy-1,2-O-isopropyliden-α-D-gluco-hexodialdo-1,4-furanose :

21 g 5-Acetamido-3-O-benzyl-5-desoxy-1,2-O-isopropyliden-α-D-glucofuranose, 54 ml Dimethylsulfoxid, 15 ml Benzol, 3 g Phosphorsäure und 37,5 g Dicyclohexylcarbodiimid werden unter Eiskühlung zusammengegeben und 3 h bei 20-25 °C gerührt. Zur Aufarbeitung wird langsam mit 12 g Oxalsäure versetzt, 30 min. gerührt, der Niederschlag abgesaugt, mit Essigester gewaschen das Filtrat mit 50 ml

gesättigter Natriumhydrogencarbonat-Lösung gewaschen und die wäßrige Phase dreimal mit je 50 ml Essigsäureethylester gewaschen. Die vereinigten Essigester-Extrakte werden über Natriumsulfat getrocknet, zweimal mit gesättigter Natriumchlorid-Lösung gewaschen, über Natriumsulfat zweimal getrocknet und im Vakuum bei 20 °C eingedampft. Das erhaltene Rohprodukt (19 g) wird sofort weiter umgesetzt.

e) 5-Acetamido-3-O-benzyl-5,7-didesoxy-1,2-O-isopropyliden-D(L)-glycero-α-D-glucohepto-1,4-furanose :

Zu 16,7 g Magnesiumspäne in 50 ml wasserfreiem Ether läßt man 39 ml Methyljodid in 300 ml wasserfreiem Ether so hinzutropfen, daß der Ether leicht siedet und kocht anschließend 30 min. unter Rückfluß. Zu dieser Lösung tropft man bei 20-25 °C 19 g rohe Acetamido-3-O-benzyl-5-desoxy-1,2-O-isopropyliden-α-D-gluco-hexodialdo-1,4-furanose in 200 ml wasserfreiem Ether hinzu und rührt über Nacht bei Raumtemperatur. Dann gibt man unter Eiskühlung vorsichtig 500 ml 20 %ige Ammoniumchlorid-Lösung hinzu, trennt die Ether-Phase ab und extrahiert dreimal mit je 100 ml Essigsäureethylester. Die vereinigten organischen Phasen werden mit Natriumhydrogencarbonat-Lösung gewaschen, über Natriumsulfat getrocknet im Vakuum eingedampft und der Rückstand aus Isopropanol umkristallisiert. Ausbeute : 3,5 g vom Schmp. 179-181 °C.

f) 5-Amino-3-O-benzyl-5,7-didesoxy-1,2-O-isopropyliden-D(L)-glycero-α-D-glucohepto-1,4-furanose :

2,4 g 5-Acetamido-3-O-benzyl-5,7-didesoxy-1,2-O-isopropyliden-D(L) glycero-α-D-glucohepto-1,4-furanose, 40 ml Ethylenglykol, 8 ml Wasser und 2 g Kaliumhydroxid werden 3 h auf 150 °C erhitzt. Nach dem Abkühlen wird mit $CO_2$ neutralisiert, die Reaktionsmischung im Hochvakuum eingedampft, der Rückstand mit heißem Ethanol digeriert, die Lösung eingedampft und der Rückstand an 250 g Kieselgel mit Ammoniak-gesättigtem-Chloroform/Ethanol 10 : 1 säulenchromatographisch gereinigt. Man erhält 1,9 g der gewünschten Verbindung als Öl.

g) 5-Acetamido-5,7-didesoxy-1,2-O-isopropyliden-D(L)-glycero-α-D-glucohepto-1,4-furanose :

20 g 5-Acetamido-3-O-benzyl-5,7-didesoxy-1,2-O-isopropyliden-D(L)-glycero-α-D-glucohepto-1,4-furanon wurden in 80 ml Methanol und 50 ml Eisessig gelöst und an 15 g 5 %igem Palladium auf Kohle unter 3,5 atm bei 30 °C 8 h hydriert. Dann wurde der Ansatz filtriert, einrotiert, der Rückstand in Essigester aufgenommen, mit Natronlauge neutralgewaschen, getrocknet und einrotiert. Man erhält 13,7 g eines Öls.

h) 5-Amino-5,7-didesoxy-1,2-O-isopropyliden-D(L)-glycero-α-D-glucohepto-1,4-furanose :

13 g 5-Acetamido-5,7-didesoxy-1,2-O-isopropyliden-D(L)-glycero-α-D-glucohepto-1,4-furanose, 24,7 g $Ba(OH)_2x8H_2O$ und 180 ml Wasser werden über Nacht unter Rückfluß gekocht. Dann gibt man 18 g Ammonium-hydrogencarbonat hinzu, rührt 2 h bei Raumtemperatur saugt den Niederschlag ab, wäscht ihn mit Wasser, engt das Eluat ein, gibt es auf eine Säule mit 250 ml stark basischem Ionenaustauscher (Lewatit n 500) und eluiert mit Wasser. Nach dem Einrotieren wird der Rückstand aus Chloroform umkristallisiert. Ausbeute : 8,5 g, Fp. : 127-131 °C.

i) 5-Amino-5,7-didesoxy-1,2-O-isopropyliden-D(L)-glycero-α-D-glucohepto-1,4-furanose :

Zu 1,9 g 6-Amino-3-O-benzyl-5,7-didesoxy-1,2-O-isopropyliden-D(L)-glycero-α-D-glucohepto-1,4-furanose in 100 ml flüssigem Ammoniak gibt man 4 g Natrium und rührt bei −70 °C über Nacht. Dann gibt man 6 g Ammoniumchlorid und 250 ml Methanol hinzu, läßt auf Raumtemperatur erwärmen, filtriert die Salze ab, dampft das Filtrat ein und chromatographiert den Rückstand an 80 g Kieselgel mit einem Gemisch aus Essigester/Methanol/konzentriertem wäßrigem Ammoniak 100 : 60 : 2. Das Eluat wird eingedampft, in heißem Isopropanol aufgenommen, filtriert und das Produkt mit der dreifachen Menge Petrolether gefällt. Ausbeute : 0,6 g.

j) 5-Amino-5,7-didesoxy-D(L)-glycero-D-glucoheptosebisulfit-Addukt :

In eine Lösung von 320 mg 5-Amino-5,7-didesoxy-1,2-O-isopropyliden-D(L)-glycero-α-D-glucohepto-1,4-furanose in 2 ml Wasser leitet man 20 h bei Raumtemperatur und anschließend 20 h bei 40 °C Schwefeldioxid ein. Dann gibt man 20 ml Methanol hinzu, worauf das gewünschte Produkt auskristallisiert. Ausbeute 200 mg vom Schmp. 128-130 °C.

k) 5-Amino-5,7-didesoxy-D(L)-glycero-O-glucoheptose :

120 mg 5-Amino-5,7-didesoxy-D(L)-glycero-D-gluco-heptose-bisulfit-Addukt werden in 5 ml Wasser gelöst, mit stark basischen Ionenaustauschern versetzt, 30 min. gerührt, filtriert, mit Wasser gewaschen

und im Vakuum bei 20 °C eingedampft. Ausbeute 70 mg.

l) 1,5,7-Tridesoxy-1,5-imino-D(L)-glycero-D-glucoheptit :

120 mg 5-Amino-5,7-didesoxy-D(L)-glycero-D-glucoheptose-bisulfit-Addukt werden in 15 ml Wasser gelöst, mit 173 mg Bariumhydroxid × 8 $H_2O$ und 400 mg Raney-Nickel versetzt und 7 h unter 3 atm bei Raumtemperatur hydriert. Dann wird die Reaktionsmischung filtriert, im Vakuum eingedampft und der Rückstand an 20 g Kieselgel mit Ether/Methanol/konz. wäßr. Ammoniak (5 : 6 : 2) säulenchromatographisch gereinigt. Ausbeute : 40,7 mg Öl, das innerhalb einiger Stunden kristallisiert. Schmp. 165-6 °C.

2. a) 2-α-Hydroxyethyl-3,4,5-trihydroxypiperidin

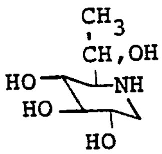

2,0 g N-Benzoyl-2-α-hydroxyethyl-3,4,5-tri-O-benzyl-3,4,5-trihydroxypiperidin in 6 ml absolutem Tetrahydrofuran werden bei −70 °C langsam zu einer Lösung von 2,0 g Natrium in 13,5 ml flüssigem Ammoniak und 5 ml absolutem Tetrahydrofuran getropft. Es wurde 4 h bei −70 °C und 1 h bei −40 °C gerührt. Dann werden 5 g $NH_4Cl$ zugegeben und der Ammoniak über Nacht abgedampft. Der Rückstand wurde mit Ethanol ausgerührt, die Salze wurden abgesaugt und die Lösung zur Trockne eingeengt. Der Rückstand wurde über eine mit Kieselgel gefüllte Säule chromatographiert. Zuerst wurde mit $CHCl_3/CH_3OH$ 4 : 1 und anschließend mit Ether/$CH_3OH$/25 %igem $NH_3$ 5 : 6 : 2 eluiert. Es wurden 150 mg Rohprodukt erhalten. Dies wurde zur weiteren Reinigung auf eine mit Amberlite IR 120 ($H^{\oplus}$-Form) gefüllte Säule aufgetragen. Es wurde zunächst mit Wasser dann mit 2 %igem Ammoniak eluiert. Ausbeute : 100 mg 2-α-Hydroxyethyl-3,4,5-trihydroxypiperidin als Harz.

b) N-Benzoyl-2-α-hydroxyethyl-3,4,5-tri-O-benzyl, 3,4,5-trihydroxypiperidin

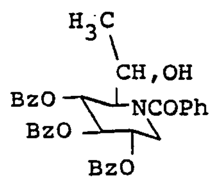

2,6 g N-Benzoyl-3,4,5-tri-O-benzyl-3,4,5-trihydroxypiperidin-2-aldehyd in 25 ml Ether wurden bei Raumtemperatur zu einer Grignard-Lösung aus 1,03 g Mg-Spänen und 2,26 ml $CH_3J$ in 5 ml absolutem Ether getropft. Es wurde 2 h unter Rückfluß erwärmt. Anschließend wurde mit $NH_4Cl$-Lösung zersetzt und mit verdünnter HCl angesäuert. Die Etherphase wurde abgetrennt, es wurde 3 mal mit Ether extrahiert. Die vereinigten Etherlösungen wurden getrocknet und eingeengt. Der Rückstand wurde mit Chloroform als Fließmittel über eine Kieselgelsäule chromatographiert. Ausbeute : 2 g N-Benzoyl-2-α-hydroxy-ethyl-3,4,5-tri-O-benzyl-3,4,5-trihydroxypiperidin als Harz.

c) N-Benzoyl-3,4,5-tri-O-benzyl-3,4,5-trihydroxy-piperidin-2-aldehyd

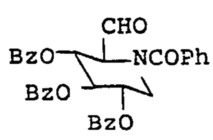

Zu 3,125 g Dicyclohexylcarbodiimid in 4,4 ml absolutem Dimethylsulfoxid und 2,5 ml Benzoyl gab man bei 20 °C unter Rühren 2,685 g N-Benzoyl-2-hydroxymethyl-3,4,5-tri-O-benzyl-3,4,5-trihydroxypiperidin und 0,25 g kristalline Orthophosphorsäure. Man hielt die Temperatur auf Raumtemperatur und rührte 3 Stunden nach. Anschließend gab man 1 g Oxalsäure hinzu und nach 30 Minuten 25 ml Essigester. Der Niederschlag wurde abgetrennt und mit Essigester nachgewaschen. Die vereinigten Essigesterlösungen wurden zunächst mit gesättigter $NaHCO_3$-Lösung und anschließend mit gesättigter Kochsalzlösung gewaschen. Die Essigesterlösung wurde über $MgSO_4$ getrocknet und das Lösungsmittel entfernt. Man erhält 2,6 g N-Benzoyl-3,4,5-tri-O-benzyl-3,4,5-trihydroxypiperidin-2-aldehyd als Harz.

d) N-Benzoyl-2-hydroxymethyl-3,4,5-tri-O-benzyl-3,4,5-trihydroxypiperidin

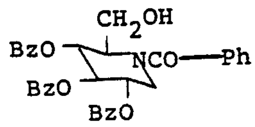

1,84 g N,7-O-Cyclocarbonato-2-hydroxymethyl-3,4,5-tri-O-benzyl-3,4,5-trihydroxypiperidin in 25 ml absolutem THF wurden zu einer Grignard-Lösung aus 1,03 g Mg-Spänen und 6,28 g Brombenzol in 5 ml absolutem THF getropft und 2 h bei 40 °C gerührt. Anschließend wurde auf 100 ml Eiswasser gegeben und mit $NH_4Cl$ und verdünnter HCl neutral gestellt. Es wurde mit $CHCl_3$ extrahiert, die Chloroformlösung wurde getrocknet und eingeengt. Zur Kristallisation wurde der Rückstand mit Ether versetzt. Ausbeute : 1,2 g N-Benzoyl-2-hydroxymethyl-3,4,5-tri-O-benzyl-3,4,5-trihydroxypiperidin vom Fp. : 104-106 °C.

28 g N-Benzoyl-7-O-trityl-3,4,5-tri-O-benzyl-2-hydroxymethyl-3,4,5-trihydroxypiperidin wurden in 200 ml 80 %iger Essigsäure gelöst und 4 h auf 60-70 °C erhitzt. Nach dem Abkühlen wurde ausgefallenes Triphenylcarbinol abgesaugt. Die Mutterlauge wurde im Vakuum eingeengt und der Rückstand mit Methanol versetzt. Das ausgefallene Triphenylcarbinol wurde abgesaugt und die Mutterlauge erneut zur Trockne eingeengt. Der Rückstand wurde über eine mit Kieselgel gefüllte Säule chromatographiert. Es wurde zunächst mit $CHCl_3$, anschließend mit $CHCl_3$/MeOH 98 : 2 eluiert. Ausbeute 11,3 g N-Benzoyl-2-hydroxymethyl-3,4,5-tri-O-benzyl-3,4,5-trihydroxypiperidin vom Fp. 106 °C.

N,7-O-Cyclocarbamato-2-hydroxymethyl-3,4,5-tri-O-benzyl-3,4,5-trihydroxypiperidin

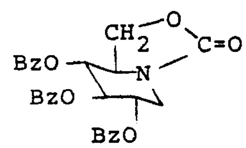

9,8 g KOH-Pulver und 2,9 g N,7-O-Cyclocarbamato-2-hydroxymethyl-3,4,5-trihydroxypiperidin in 100 ml DMSO wurden 30 Minuten unter Rühren auf 60 °C erhitzt. Dann tropfte man bei 60 °C 17,6 ml Benzylchlorid zu. Es wurde weitere 30 Minuten bei 60° gerührt. Anschließend wurde das DMSO an der Ölpumpe abdestilliert. Die Rückstand wurde auf Eiswasser gegeben und die Wasserphase mit konz. HCl neutral gestellt. Dann wurde mit Chloroform extrahiert. Die Chloroformlösung wurde getrocknet und eingeengt. Der Rückstand wurde über eine mit Kieselgel gefüllte Säule chromatographiert (Elutionsmittel : $CHCl_3$/MeOH 40 : 1). Ausbeute : 4,6 g N,7-O-Cyclocarbamato-2-hydroxymethyl-3,4,5-tri-O-benzyl-3,4,5-trihydroxypiperidin. Beim Anreiben mit Cyclohexan oder mit wenig Methanol wird die Substanz kristallin. Fp. : 104-105 °C.

N,7-O-Cyclocarbamato-2-hydroxymethyl-3,4,5-trihydroxypiperidin.

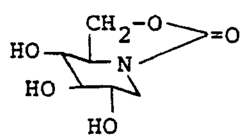

Zu einem Gemisch von 2,4 g 1-Desoxynojirimycin und 3,2 g fein gepulvertem $K_2CO_3$ in 50 ml absolutem DMF wurden bei 15 °C 2,58 ml Chlorameisensäureethylester unter Rühren langsam zugetropft. Es wurde 1 Stunde bei Raumtemperatur gerührt und anschließend 3 h auf 100 °C erwärmt. Dann wurde von den Salzen abgesaugt, die DMF-Lösung wurde im Vakuum eingeengt und der Rückstand mit Ethanol kristallisiert. Ausbeute : 2 g N,7-O-Cyclocarbamato-2-hydroxymethyl-3,4,5-trihydroxypiperidin.

Zur weiteren Reinigung kann die Substanz aus Ethanol/wenig Wasser umkristallisiert werden. Fp. : 218 °C.

N-Benzoyl-7-O-trityl-2-hydroxymethyl-3,4,5-tri-O-benzyl-3,4,5-trihydroxypiperidin

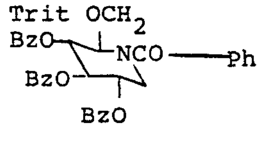

1,35 g 80 %iges NaH wurden mit 50 ml n-Hexan verrührt. Das n-Hexan wurde abdekantiert und durch 50 ml absolutes DMSO ersetzt. Anschließend wurde unter $N_2$ 1 h auf 60-70 °C erwärmt. Nach dem Abkühlen tropfte man 5,1 g N-Benzoyl-7-O-trityl-2-hydroxymethyl-3,4,5-trihydroxypiperidin in 30 ml absolutem DMSO zu und ließ 1 h bei Raumtemperatur rühren. Dann wurde 4,2 g Benzylchlorid in 25 ml DMSO zugetropft und es wurde über Nacht gerührt. Das Reaktionsgemisch wurde mit 300 ml $CH_2Cl_2$ versetzt

und mit 200 ml $H_2O$ ausgeschüttelt. Die $CH_2Cl_2$-Phase wurde noch 2 mal mit Wasser gewaschen, über $Na_2SO_4$ getrocknet und im Vakuum eingeengt. Ausbeute : 6,5 g an rohem N-Benzoyl-7-O-trityl-2-hydroxymethyl-3,4,5-tri-O-benzyl-3,4,5-trihydroxypiperidin. Das Rohprodukt wird in die nächste Reaktionsstufe eingesetzt.

N-Benzoyl-7-O-trityl-2-hydroxymethyl-3,4,5-trihydroxypiperidin

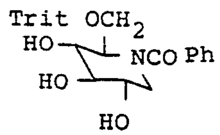

63,9 g N-Benzoyl-2-hydroxymethyl-3,4,5-trihydroxypiperidin und 79,9 g Tritylchlorid in 250 ml absolutem Pyridin wurden 24 h bei Raumtemperatur gerührt. Anschließend wurden weitere 80 g Tritylchlorid zugegeben und es wurde noch einmal 48 h lang gerührt. Der Niederschlag wurde abgesaugt und die Mutterlauge im Vakuum eingeengt. Der Rückstand wurde in $CHCl_3$ gelöst und die Chloroformlösung mit $H_2O$ gewaschen. Die Chloroformphase wurde mit $Na_2SO_4$ getrocknet und im Vakuum eingeengt. Der Rückstand wurde anschließend in wenig Toluol aufgenommen. Durch Eintropfen der Toluollösung in einen großen Überschuß Cyclohexan wurde das Reaktionsprodukt ausgefällt. Der Niederschlag wurde abgesaugt und getrocknet. Ausbeute : 90 g rohes N-Benzoyl-7-O-trityl-2-hydroxymethyl-3,4,5-trihydroxypiperidin. Das Rohprodukt kann durch Verreiben mit Ether oder durch Umkristallisation aus wenig Toluol weiter gereinigt werden. Fp. : 185-187 °C.

N-Benzoyl-2-hydroxymethyl-3,4-5-trihydroxypiperidin

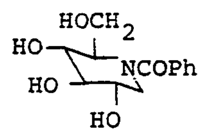

Zu einer Lösung von 30 g 1-Desoxynojirimycin in 120 ml $H_2O$, 350 ml $CH_3OH$ und 30 ml Triethylamin wurden bei 30-35 °C 27 ml Benzoylchlorid in 300 ml Essigester getropft. Es wurde 1 Stunde bei Raumtemperatur nachgerührt und anschließend werden bei 30-35 °C weitere 15 ml Triethylamin und 13, 5 ml Benzoylchlorid in 150 ml Essigester zugetropft. Nach 1-stündigem Rühren wurde das Reaktionsgemisch im Vakuum zur Trockne gebracht. Der Rückstand wurde in Wasser aufgenommen und mit Ether extrahiert. Die wäßrige Phase wurde erneut im Vakuum zur Trockne eingeengt und der Rückstand mit Aceton verrührt. Das ausgefallene Triethylaminhydrochlorid wird abgesaugt. Durch erneutes Einengen der Acetonlösung und Aufnehmen des Rückstands in wenig Aceton wurde restliches Triethylaminhydrochlorid abgetrennt. Nach Entfernen des Lösungsmittels erhält man das Produkt als Harz. Nach gründlicher Trocknung kann dieses Harz in die nächste Stufe eingesetzt werden. Ausbeute : 56 g an rohem N-Benzoyl-3,4,5-trihydroxypiperidin. Nach längerem Stehen kristallisiert die Verbindung aus Aceton. Fp. : 159 °C.

In Analogie zu den Beispielen 2a und 2b wurden hergestellt :

## Beispiel 3

(mit Aethylmagnesiumjodid bei Raumtemperatur)

2-α-Hydroxypropyl-3,4,5-trihydroxypiperidin

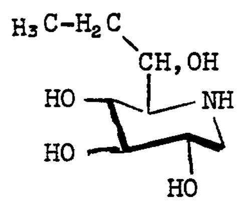

Das nicht kristalline Produkte wurde durch ein Protonenresonanzspektrum bei 250 MHz charakterisiert.

Rf-Wert : 0,52

Rf-Wert für 1-Desoxynojirimycin : 0,31

[DC-Fertigkplatten, Kieselgel 60 F 254, Merck (Darmstadt) ; Fließmittel : $CHCl_3$/MeOH/25 %iges $NH_3$, 4 : 3 : 1].

Beispiel 4

(mit n-Butyllithium bei −70 °C)

2-α-Hydroxypentyl-3,4,5-trihydroxypiperidin

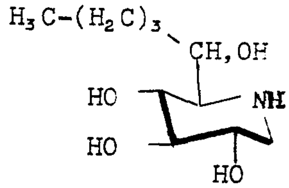

Rf-Wert : 0,65 (Chromatographiebedingungen wie in Beispiel 3).

Beispiel 5

(mit Phenylmagnesiumbromid bei −20 °C)

2-α-Hydroxybenzyl-3,4,5-trihydroxypiperidin

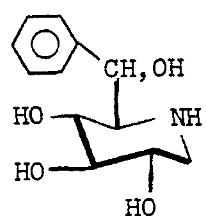

Rf-Wert : 0,82 (Chromatographiebedingungen wie in Beispiel 3).

**Ansprüche**

1. 2-Hydroxyalkyl-3,4,5-trihydroxypiperidine der allgemeinen Formel I

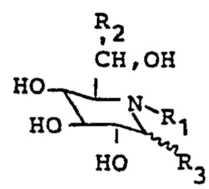

(I)

worin

$R_1$ Wasserstoff,

$R_2$ gegebenenfalls durch Hydroxy, $C_1$-$C_4$-Alkoxy oder Phenyl, das seinerseits durch $C_1$-$C_4$-Alkyl, $C_1$-$C_4$-Alkoxy, Halogen, Nitro oder Cyan substituiert sein kann, substituiertes $C_1$-$C_{10}$-Alkyl, $C_2$-$C_6$-Alkenyl, $C_4$-$C_{10}$-Alkandienyl oder gegebenenfalls durch $C_1$-$C_4$-Alkyl, $C_1$-$C_4$-Alkoxy, Halogen, Nitro oder Cyan substituiertes Phenyl und

$R_3$ Wasserstoff, Sulfo oder Hydroxy bedeuten.

2. Verbindungen gemäß der allgemeinen Formel I nach Anspruch 1, worin

$R_2$ $C_1$-$C_6$-Alkyl, Allyl, Benzyl oder Phenyl

bedeutet.

3. Verbindungen gemäß der allgemeinen Formel I nach Anspruch 1 oder 2, worin

$R_2$ Methyl oder Ethyl und

$R_3$ Wasserstoff bzw.

$R_2$ Methyl und

$R_3$ Sulfo oder Hydroxy bedeuten.

4. Verfahren zur Herstellung von Verbindungen nach Anspruch 1, dadurch gekennzeichnet, daß man Verbindungen der allgemeinen Formel II

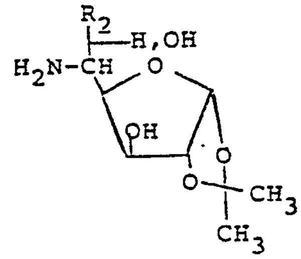

worin $R_2$ die obengenannte Bedeutung hat, mit Schwefeldioxid umsetzt, die entstehenden Verbindungen der allgemeinen Formel I, worin $R_3$ $SO_3H$ bedeutet, gegebenenfalls mit Basen oder basischen Ionenaustauschern zu Verbindungen der allgemeinen Formel I, worin $R_3$ OH bedeutet, umsetzt, und diese gegebenenfalls zu Verbindungen der allgemeinen Formel I, worin $R_3$ Wasserstoff bedeutet, reduziert.

5. Verfahren zur Herstellung von Verbindungen nach Anspruch 1 der allgemeinen Formel I

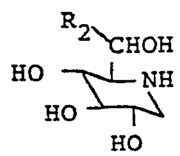

dadurch gekennzeichnet, daß man einen Aldehyd der allgemeinen Formel IV

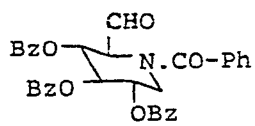

Bz = Benzyl
Ph = Phenyl

mit einer Grignard-Verbindung $R_2MgX$, in der $R_2$ die in Anspruch 1 angegebene Bedeutung hat und X Halogen darstellt, umsetzt und das so erhaltene Grignardadditionsprodukt mit Natrium in flüssigem Ammoniak zum Endprodukt umsetzt.

6. Arzneimittel, gekennzeichnet durch einen Gehalt an einer Verbindung gemäß Ansprüchen 1 bis 3.

**Claims**

1. 2-Hydroxyalkyl-3,4,5-trihydroxypiperidines of the general formula I

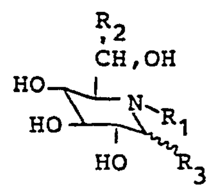

(I)

wherein

$R_1$ denotes hydrogen,

$R_2$ denotes $C_1$-$C_{10}$-alkyl, $C_2$-$C_6$-alkenyl or $C_4$-$C_{10}$-alkanedienyl which is optionally substituted by hydroxyl, $C_1$-$C_4$-alkoxy or phenyl, which can in turn be substituted by $C_1$-$C_4$-alkyl, $C_1$-$C_4$-alkoxy, halogen, nitro or cyano, or phenyl which is optionally substituted by $C_1$-$C_4$-alkyl, $C_1$-$C_4$-alkoxy, halogen, nitro or cyano and

$R_3$ denotes hydrogen, sulpho or hydroxyl.

2. Compounds of the general formula I according to Claim 1, wherein

$R_2$ denotes $C_1$-$C_6$-alkyl, allyl, benzyl or phenyl.

3. Compounds of the general formula I according to Claim 1 or 2, wherein

$R_2$ denotes methyl or ethyl and

$R_3$ denotes hydrogen, or

$R_2$ denotes methyl and

$R_3$ denotes sulpho or hydroxyl.

4. Process for the preparation of compounds according to Claim 1, characterised in that compounds of the general formula II

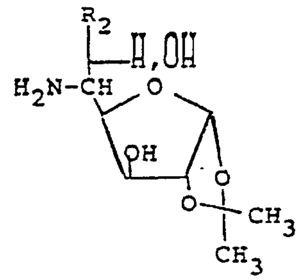

(II)

wherein $R_2$ has the abovementioned meaning, are reacted with sulphur dioxide, the resulting compounds of the general formula I wherein $R_3$ denotes $SO_3H$ are reacted, if desired, with bases or basic ion exchangers to give compounds of the general formula I wherein $R_3$ denotes OH, and these compounds are reduced, if desired, to give compounds of the general formula I wherein $R_3$ denotes hydrogen.

5. Process for the preparation of compounds according to Claim 1 of the general formula I

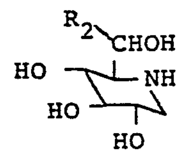

characterised in that an aldehyde of the general formula IV

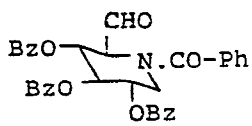

Bz = benzyl
Ph = phenyl

is reacted with a Grignard compound $R_2MgX$, in which $R_2$ has the meaning indicated in Claim 1 and X represents halogen, and the Grignard addition product thus obtained is reacted with sodium in liquid ammonia to give the end product.

6. Medicaments, characterised in that they contain a compound according to Claims 1 to 3.

**Revendications**

1. 2-Hydroxyalkyl-3,4,5-trihydroxypipéridines de formule générale I

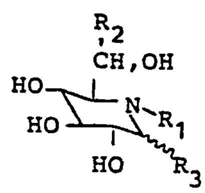

(I)

dans laquelle

$R_1$ est l'hydrogène,

$R_2$ est un groupe alkyle en $C_1$ à $C_{10}$ éventuellement substitué par un radical hydroxy, alkoxy en $C_1$ à $C_4$ ou phényle qui peut être substitué de son côté par un radical alkyle en $C_1$ à $C_4$, alkoxy en $C_1$ à $C_4$, un halogène, un radical nitro ou cyano, un groupe alcényle en $C_2$ à $C_6$, un groupe alcanediényle en $C_4$ à $C_{10}$ ou un groupe phényle éventuellement substitué par un radical alkyle en $C_1$ à $C_4$, alkoxy en $C_1$ à $C_4$, un halogène, un radical nitro ou cyano et

$R_3$ représente l'hydrogène, un groupe sulfo ou un groupe hydroxy.

2. Composés de formule générale I suivant la revendication 1, dans lesquels

$R_2$ est un groupe alkyle en $C_1$ à $C_6$, allyle, benzyle, ou phényle.

3. Composés de formule générale I suivant la revendication 1 ou 2 dans lesquels

$R_2$ est le groupe méthyle ou éthyle et

$R_3$ est l'hydrogène ou

$R_2$ est le groupe méthyle et

$R_3$ est le groupe sulfo ou hydroxy.

4. Procédé de production de composés suivant la revendication 1, caractérisé en ce qu'on fait réagir des composés de formule générale II

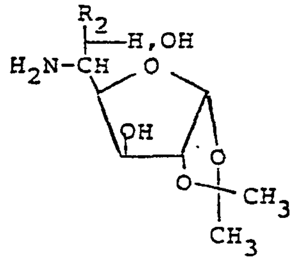

(II)

dans laquelle $R_2$ a la définition mentionnée ci-dessus, avec l'anhydride sulfureux, on fait réagir les composés produits de formule générale I, dans laquelle $R_3$ désigne $SO_3H$, éventuellement avec des bases ou des échangeurs d'ions basiques pour former des composés de formule générale I dans laquelle $R_3$ représente OH, et on réduit ces composés éventuellement en composés de formule générale I dans laquelle $R_3$ désigne l'hydrogène.

5. Procédé de production de composés suivant la revendication 1 de formule générale I

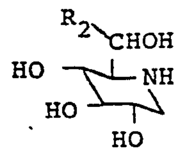

caractérisé en ce qu'on fait réagir un aldéhyde de formule générale IV

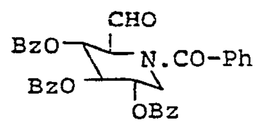

Bz = Benzyle
Ph = Phényle

avec un composé de Grignard $R_2MgX$ dans lequel $R_2$ a la définition indiquée dans la revendication 1 et X représente un halogène, et on fait réagir le produit d'addition de Grignard ainsi obtenu avec du sodium dans l'ammoniac liquide pour obtenir le produit final.

6. Médicament, caractérisé par une teneur en un composé suivant les revendications 1 à 3.